# EUROPEAN PATENT SPECIFICATION

(11) **EP 0 721 457 B1**
(45) Date of publication and mention of the grant of the patent: **06.05.1999**
(21) Application number: 93920816.1
(22) Date of filing: 24.09.1993
(51) Int. Cl.: C07H 21/00, A61K 31/70, C12P 19/34

(54) **2',5'-OLIGOADENYLATE-2',3'-CYCLOPHOSPHATES and 2',5'-OLIGOADENYLATES with antipapilloma virus activity**
Gegen Papillomaviren wirksame 2',5'-OLIGOADENYLAT-2',3'-CYCLOPHOSPHATE und 2',5'-OLIGOADENYLATE
2',5'-OLIGOADENYLATE-2',3'-CYCLOPHOSPHATES et 2',5'-OLIGOADENYLATES avec activité antipapillome

(43) Date of publication of application: 17.07.1996
(73) Proprietor: Budowsky, Edward I., Brookline MA 02146 (US); Gavrilov, Alexander E., Moscow (RU)
(72) Inventor: Budowsky, Edward I., Brookline MA 02146 (US); Gavrilov, Alexander E., Moscow (RU); PIVASYAN, Arman, D., New Haven, CT 06511 (US); ZHELKOWSKY, Alexander, Beverly MA 01915 (US)
(74) Representative: Armitage, Ian Michael
(86) International application number: PCT/EP93/02596
(87) International publication number: WO 95/08555

(56) References cited:
- US-A- 4 378 352
- NUCLEIC ACIDS RES. (NARHAD,03051048);84; VOL.12 (7); PP.3257-70, KYOTO UNIV.;GRAD. SCH. ENG.; KYOTO; 606; JAPAN (JP) SHIMIDZU T ET AL 'A simple and convenient synthesis of 3'-5'- or 2'-5'-linked oligoribonucleotide by polymerization of unprotected ribonucleoside using phosphorus tris-azole'
- BIOCHIMICA ET BIOPHSICA ACTA, Bd.240, 1971, AMSTERDAM, NL Seiten 463 - 471 RENZ M. ET AL 'Catalysts for the polymerization of adenosine cyclic 2',3'-phosphate on a poly (U) template'
- BIOCHEMISTRY (BICHAW);77; VOL.16 (3); PP.493-8, OSAKA UNIV.;FAC. PHARM. SCI.; SUITA; JAPAN UESUGI S ET AL 'Polynucleotides. 41. Synthesis and template-directed polymerization of adenylyl(3'-5')adenosine cyclic 2',3'-phosphate'

## Description

Die Erfindung betrifft am 3'-Ende eine Cyclophosphatgruppe und am 5'-Ende eine freie OH-Gruppe aufweisende 2',5'-Oligoadenylate zur pharmazeutischen Verwendung, ein sie enthaltendes pharmazeutisches Präparat und die Verwendung dieser Verbindungen zur Behandlung äußerer Papillomatosen.

Die Erfindung betrifft 2',5'-Oligoadenylat-2',3'-cyclophosphate der allgemeinen Formel 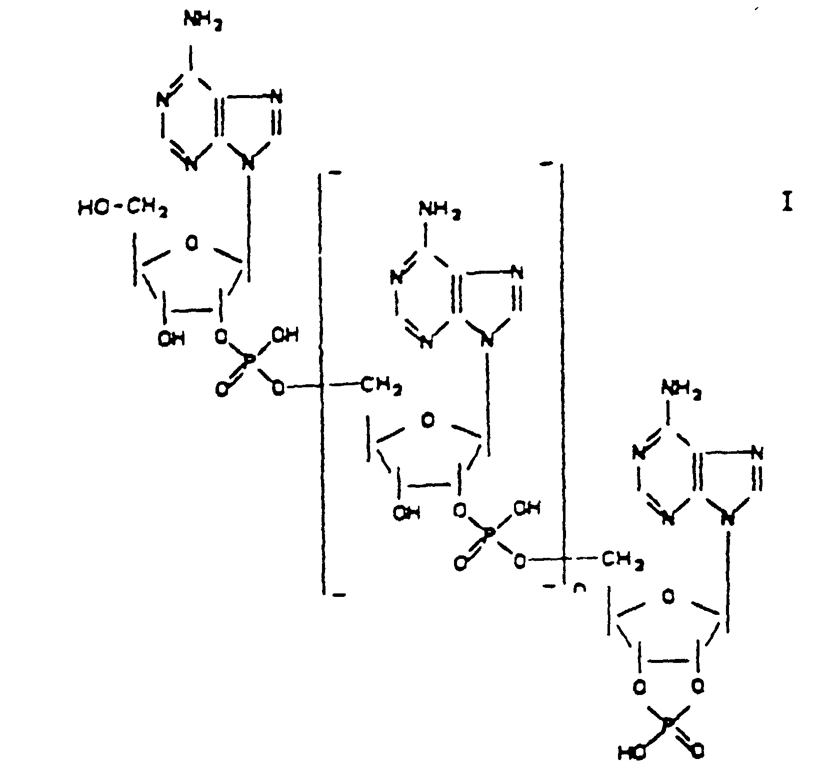 worin 0 ≤ n ≤ 10, insbesondere ≥ 0 bis 10, vorzugsweise 1 oder 2, zur pharmazeutischen Verwendung.

Insbesondere die Verbindungen mit n = 1 oder 2 können vorteilhaft für medizinische Zwecke, und zwar für die topische Behandlung von Haut und Epithelläsionen verwendet werden, die durch Papillomaviren verursacht werden.

Durch Papillomaviren der Familie Papovaviridae verursachte Papillomatosen sind weit verbreitete Infektionskrankheiten bei Mensch und Tier. Gegenwärtig sind mehr als 60 Typen menschlicher Papillomaviren bekannt.

All diese Viren weisen ähnliche Struktur auf. Ihr Genom stellt jeweils eine doppelsträngige kovalent geschlossene ringförmige DNA mit 8.000 Basenpaaren dar, welche die Virionenproteine und die für die interzelluläre Entwicklung des Virus erforderlichen Proteine kodiert. In der infizierten Zelle wird das Genom der Papillomaviren im Verlaufe vieler Generationen in Form von Episomen (Dutzende Kopien pro Zelle) redupliziert. Die Bildung der reifen Virionen erfolgt erst in den Zellen auf der Endstufe der Differenzierung.

Unter persistenten Bedingungen werden nur die ersten Gene des Papillomavirusgenoms exprimiert, wobei sie eine Veränderung des Zellphänotyps verursachen und auf diese Weise zur Bildung von Papillomatosen führen. In den infizierten Zellen kann ein bestimmter Teil des Virusgenoms mit einer vom Virustyp und anderen Faktoren abhängigen Wahrscheinlichkeit in das Zellgenom eingebaut werden, was dann die Malignisierung auslösen kann. Es ist bekannt, daß eine erhebliche Zahl von menschlichen Tumoren das Ergebnis der Malignisierung von Papillomatosen ist. Diese stellen somit eine Folge persistenter latenter Virusinfektionen dar.

Auf sexuellem Wege übertragene Anogenitalpapillomatosen sind dabei, was die Malignisierung betrifft, am gefährlichsten (s. M. Spitzer, Obstet. Gynecol., 1989, vol. 73, N3, S. 303-307;, H. zur Hausen, A. Schneider, The Role of Papillomaviruses in Human Anogenital Cancer, in: The Papovaviridae (N.P. Alzman ed.), 1987, vol. 2, S. 245-263; H. zur Hausen, Papillomaviruses as Carcinomaviruses, in: Adv. in Virus Oncology (G. Klein ed.), 1989, vol. 8, S. 1-26).

Da Papillomatosen leicht zu diagnostizierende präkanzeröse Erkrankungen sind, kann die Entwicklung vieler Tumore durch Behandlung gutartiger Papillomatosen verhindert werden, d.h. bevor es zu einer malignen Umwandlung der infizierten Zellen kommt.

Gegenwärtig sind die wichtigsten Methoden zur Behandlung von Papillomatosen die chirurgische Entfernung der Papillome sowie die Nekrotisierung durch Elektro-, Kryo- oder Laserkauterisation (s. Virus infections. Etiology, epidemiology, clinics, pathogenesis and diagnosis. Rep. Col. of Scient. Public., Sverdlovsk, 1985 (in Russisch)). Zu diesem Zweck verwendet man flüssigen Sauerstoff, Säuren und deren Gemische (Salpeter-, Oxal-, Milchsäure usw.), welche die Nekrose der umgebenden gesunden Gewebe verursachen, an der Applikationsstelle zur Narbenbildung und häufig zu Rückfällen sowie zum Auftreten neuer Papillome nahe der Stelle, an der die alten entfernt wurden, führt (s. S.A. Bashi. Cryoterapia versus podophyllin in the treatment of genital warts. Int. J. Dermatol., 1985, vol. 24, N 8, s. 535-536).

Die Wirksamkeit medizinischer Methoden zur Behandlung von Papillomatosen unter Verwendung von Podophyllotoxin und Interferon ist gering und geht außerdem Hand in Hand mit starken Neben- bzw. Nachwirkungen, selbst wenn mit therapeutischen Dosen gearbeitet wird.

Die biologische Aktivität von Podophyllin läßt sich durch ihre mit Colchicin vergleichbare antimitotische Wirkung erklären. Seine Anwendung verursacht häufig lokale Reaktionen (Entzündungen, allergische Kontaktdermatosen, gelegentliche Hauterosionen usw.) sowie unerwünschte Nachwirkungen wie periphere Neuropathie, Tachipnoe, Hämaturie und Abortus spontaneus (s. K.R. Beutner. Podophyllotoxin in the treatment of Genital Human Papillomavirus Infections. Seminars in Dermatology, 1987, vol. 6, N1, S. 10-18).

Die Applikation von Interferon im Falle von Papillomatosen zeigt nur geringe Wirkung und die für diese Behandlung eingesetzten Dosen können zur Suppression des Immunsystems sowie zur Auslösung von Autoimmunerkrankungen usw. führen (s. F.G. Bruins, A.J.C. von den Brule, R. Mullnik, G.M.M. Walboomers, C.J. Meijer, R. Willemze. J. Invest. Dermatol., 1989, vol. 93, N4, S. 544-545; M. Foldvan, A. Moreland, M. Nezei, ibid., S. 550; G. Gross, Roussaki, ibid., S. 553; M. Niimura, imbid., S. 567).

Synthetische Analoga von 2',5'-Oligoadenylaten (2,5 A) sind dafür bekannt, daß sie immunosuppressorische Aktivität zeigen und wurden bisher für chirurgische Transplantationen vorgeschlagen. Es wurde festgestellt, daß Oligoadenylate als Mediatoren für die durch Interferon verursachten Wirkungen weniger toxisch, spezifischer wirksam und effektiver sind als Immunsuppressoren (s. A. Kimchi et al., US-Patent 4 378 352 (1983)).

Dieselbe Wirkung wird auch durch ein eine endständige Morpholingruppe enthaltendes synthetisches 2,5 A (s. R. Torrence et al., US-Patent 4 515 781 (1985)) erzielt.

2,5 A-Analoga mit mindestens drei Adenosinfragmenten sind als aktive Inhibitoren der Virusproteinsynthese in vitro bekannt (s. Jan M. Kerr et al., US-Patent 4, 21, P, 746 (1980)).

Einige synthetische Analoga von 2,5 A-Oligo-3'-deoxyadenylaten und ihre Derivate inhibieren insbesondere in in vitro-Kulturen die Infektion und Transformation tierischer Zellen mit Herpes simplex und Epstein-Barr-Viren, sind jedoch inaktiv im Falle bereits infizierter oder transformierter Zellen (s. R.I. Suhadolnik et al., US-Patent 4 464 359 (1984); R.I. Suhadolnik et al., US-Patent 4 539 313 (1985); R.I. Suhadolnik et al., US-Patent 4 708 935 (1987)).

Möglich ist die Verwendung von 2,5 A für die Behandlung von Infektionskrankheiten, die durch Cytomegalovirus, Hepatitis-B-Virus und Varicella zoster-Viren verursacht werden (s. Europäische Patentanmeldung Nr. 121 635 (Au, No. J, Dk, Fi, Fr, Es), 1984).

Shimidzu et al. (Nucleic Acid Research, 12(7): 3257-3270, 1984) gibt eine Methode dafür bekannt, 3',5'- bzw. 2',5'-Oligoribonucleotide dadurch zu bereiten, Ribonucleotide mit Anwendung von Phosphortriazol zu polymerisieren. In diesem Schriftstück findet man keine Bekanntgebung noch Andeutung für die pharmazeutische Verwendung von irgendwelchen von diesen Verbindungen.

Renz et al. (Biocchemica et Biophysica Acta, 240: 463-471, 1971) teilt die Bildung von Mischungen von 2',5'- und 3',5'- Adenylaten nach der spontanen Polymerisierung von Adenosin-Cyclophosphat auf einer Poly(U)-Schablone mit. Keine Verwendungen von den unter diesem Zeichen bekanntgegebenen Verbindungen werden vorgeschlagen.

Aufgabe der Erfindung ist die Bereitstellung eines wirksamen Arzneimittels mit selektiver Wirkung für die Behandlung von durch Papillomaviren verursachten Haut- und Epithelläsionen.

Der Erfindungszweck wird durch Bereitstellung von 2',5'-Oligoadenylat-2',3'-cyclophosphaten der Formel I erzielt 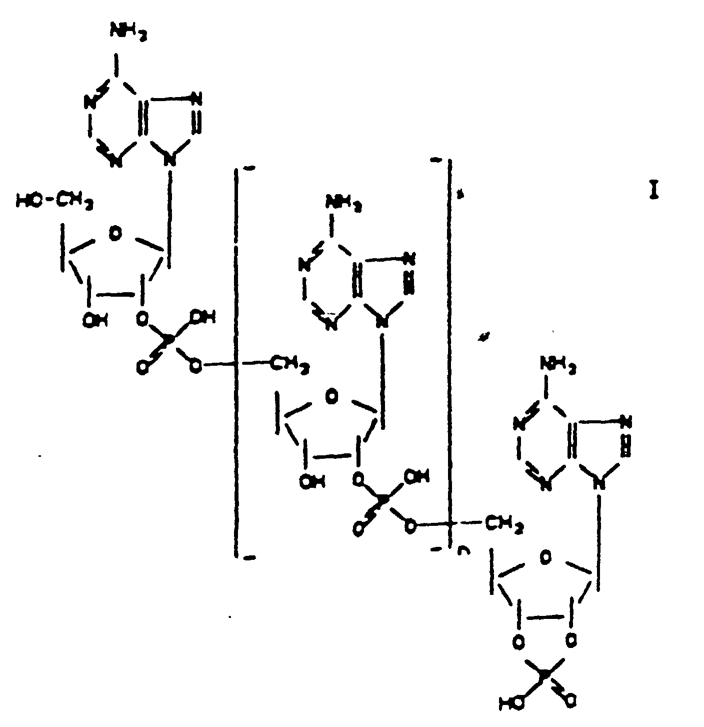 worin 0 ≤ n ≤ 10, insbesondere ≥ 0 bis 10, vorzugsweise 1 oder 2.

Gegenstand der Erfindung sind auch Verfahren zur Herstellung der neuen Verbindungen, wie sie im nachfolgenden im einzelnen beschrieben werden, ausgehend von Poly(A).

Diese Verbindungen können in an sich bekannter Weise hergestellt werden ausgehend von Poly(A) mit unregelmäßigen 2',5'- und 3',5'-Internucleotidbindungen nach einem bekannten Verfahren (s. A.M. Michelson: In the Chemistry of the Nucleosides and Nucleotides (Academic Press) 1963, S. 418 und 419) durch chemische Polymerisation von 2'(3')-Adenosinmonophsphat. Die nachfolgende Spaltung der 3',5'-Bindungen in diesem Polymer durch Ribonuklease aus B. intermedius (E.C.3.1.4.23) führt zu einem Monomer und 2',5'-Oligoadenylate von unterschiedlicher Länge mit einem endständige 2',3'-Cyclophosphatgruppen enthaltenden Gemisch.

Dieselben Ergebnisse erzielt man auch mit einem anderen zweistufigen Verfahren:
1. Spaltung der Poly(A) mit Ribonuclease T2 (oder ähnlichen Ribonucleasen), was zu einer Reihe von 2',5'-Oligoadenylaten führt, wobei jedes Oligomer ein Gemisch aus 2',3'-Cyclophosphat und 3'-Monophosphat darstellt, und
2. Behandlung dieses Gemisches mit einem 100-fachen Überschuß an BrCN in gepufferter wässeriger Lösung, was zur Umwandlung der endständigen 3'-Phosphatgruppe in die 2',3'-Cyclophosphatgruppe führt.

Diese beiden Synthesewege können schematisch wie folgt dargestellt werden:

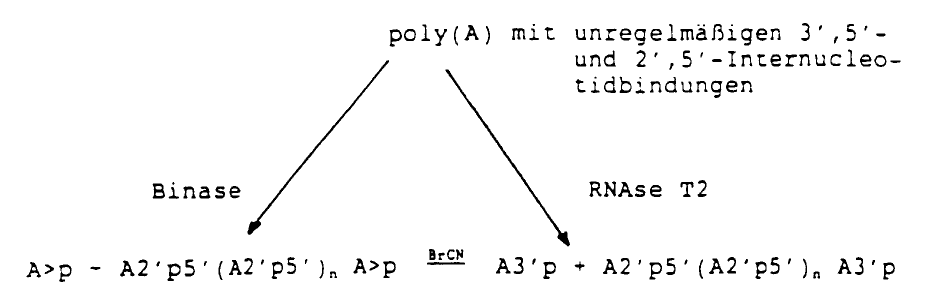

Die erhaltenen gemische aus 2',5'-Oligoadenylaten werden analysiert und die erwünschten Oligonucleotide gemäß Formel I werden durch HPLC gereinigt.

2',5'-0ligoadenylate mit einer endständigen 2',3'-Cyclophosphatgruppe als Einzelverbindungen wurden bisher nicht beschrieben. Sowohl die 2'(3')Phosphatgruppen als auch die 2',3'-Cyclophosphatgruppen in den 2',5'-Oligoadenylaten verhindern wirksam die Hydrolyse dieser Verbindungen mit Zellenzymen. Die Wirksamkeit der 2'-3'-Cyclophosphatanaloga gegenüber den natürlichen, die 5'-Triphosphatgruppe enthaltenden 2',5'-Oligoadenylaten erklärt sich durch das höhere Zellpermeationsvermögen aufgrund der geringeren Ladung und die Beständigkeit gegenüber der Hydrolyse durch Phosphodiesterasen mit Zellenzymen.

Die erfindungsgemäßen 2',5'-Oligadenylate werden in Wasser oder wässerigen Lösungen von neutralen Salzen gelöst und 1 bis 2 mal täglich während 2 Wochen auf die Hautläsionen (gewöhnliche flache Warzen sowie Sohlenwarzen, Kondylome usw.) aufgebracht. Die Wirkstoffkonzentration beträgt 10⁻⁴ bis 10⁻⁷ M.

Infolge der Behandlung der Kondylome kommt es zum Wachstumsstillstand; eine Neubildung erfolgt nicht und die alten Kondylome werden innerhalb von 4 bis 6 Wochen in den meisten Fällen zurückgebildet.

Im Falle gewöhnlicher Warzen kommt es gegen Ende der ersten Woche zu einer Abflachung der Papeln und 2 bis 4 Wochen nach Beginn der Behandlung verschwinden sie vollständig. Patienten mit flachen Warzen sind 1 Monat nach Beginn der Behandlung vollständig geheilt.

Im Verlaufe der Behandlung von Papillomen schrumpfen nach 1,5 bis 2 Wochen die Neubildungen, und kleinere Papillome werden abgestoßen, und innerhalb 1 Monats kommt es zur vollständigen Rückbildung der Hautläsionen. In keinem einzigen Fall kommt es zur Narbenbildung. Die Heilung wird auch nicht von toxisch-allergischen Reaktionen oder anderen Nebenwirkungen begleitet.

Die Konzentration der aktiven Oligoadenylate in den Behandlungslosungen ist zwar wenigstens um zwei Größenordnungen höher als im Falle der interferoninduzierten Zellen, die Gesamtmenge an aufgebrachter Lösung liegt jedoch gewöhnlich unter 1,0 ml. Selbst wenn alle Oligoadenylate absorbiert werden, liegt deshalb die Durchschnittskonzentration in den Zellen und Körperflüssigkeiten (Blut, Urin usw.) weit unter der in den nicht induzierten Zellen. Die Konzentration an den aufgebrachten Verbindungen kann nur vorübergehend (unmittelbar nach Behandlung) höher-sein als die ihrer natürlichen Analoga in den normalen Zellen, und das auch nur in den Geweben in der unmittelbaren Umgebung der Applikationsstelle.

Die 2',5'-Oligoadenylate können durch Nucleasen und Phosphatasen, die innerhalb und außerhalb der Zellen des Organismus vorhanden sind, leicht abgebaut werden. Als Endprodukte fallen dabei Adenylsäure und Adenosin an, die übliche Zellmetabolite darstellen, deren Durchschnittskonzentration in den Zellen unter 10⁻³ M liegt.

Für die Behandlung von durch Papillomaviren induzierten Haut- und Epithelläsionen können sowohl einzelne Oligonucleotide der Formel I als auch sie enthaltende Gemische verwendet werden.

Die topische Anwendung geringer Dosen der erfindungsgemäßen 2',5'-Oligoadenylate zur Behandlung von Papillomatosen ist überaus wirksam, wobei keine negativen Hautreaktionen und auch keine systemischen Nebenwirkungen beobachtet werden. Ergebnis der Behandlung der Läsionen ist eine vollständige Rückbildung bei mehr als 80 % der Patienten. Nach einer Beobachtungsdauer von 3 Monaten sind keine Rückfälle festzustellen.

Das erfindungsgemäße Präparat kann in unterschiedlichen, für die topische Anwendung annehmbaren Formulierungen verabreicht werden. Diese stellt man her durch sorgfältiges Mischen bzw. Lösen der Einzelverbindungen oder ihrer Gemische mit einem pharmazeutisch verträglichen Träger unter Verwendung der üblichen Techniken, wobei der Träger je nach der für die Applikation erwünschten Formulierung des Präparats, d.h. auf der Außenhaut oder auf Schleimhautgeweben sehr unterschiedliche Form haben kann. Für die Herstellung der Präparate können die verschiedensten pharmazeutischen Medien verwendet werden, wie z.B. flüssige Träger wie Wasser, Dimethylsulfoxid in An- oder Abwesenheit von Alkoholen, Glycolen usw. oder in Form einer Pomade, einer Salbe oder eines Pflasters. Besonders vorteilhaft ist es, die erwähnten pharmazeutischen Präparate zur Erleichterung und Vereinheitlichung der Dosierung in einer Dosiseinheitsform zu formulieren. Der Ausdruck "Dosiseinheitsform" bezieht sich auf physikalisch diskrete Einheiten, von denen jede eine bestimmte Menge Wirkstoff enthält, die so berechnet ist, daß in Verbindung mit dem erforderlichen pharmazeutischen Träger die gewünschte therapeutische Wirkung erzielt wird. Die entsprechende Dosis für eine einzige Anwendung zur Behandlung von Papillomatosen sollte 10⁻⁹ bis 10⁻⁸ M Wirkstoff (n = 1 und/oder 2) pro Papel (3 bis 5 mm im Durchmesser) zu betragen. Die Gesamtdauer der Behandlung beträgt bis zu 30 Tagen bei täglicher Applikation.

Die folgenden Beispiele illustrieren die Herstellung von Oligonucleotidgemischen und einzelnen 2',5'-Oligoadenylaten.

Chemisch-enzymatische Synthese, Reinigung und Analyse der 2',5'-Oligoadenylate.

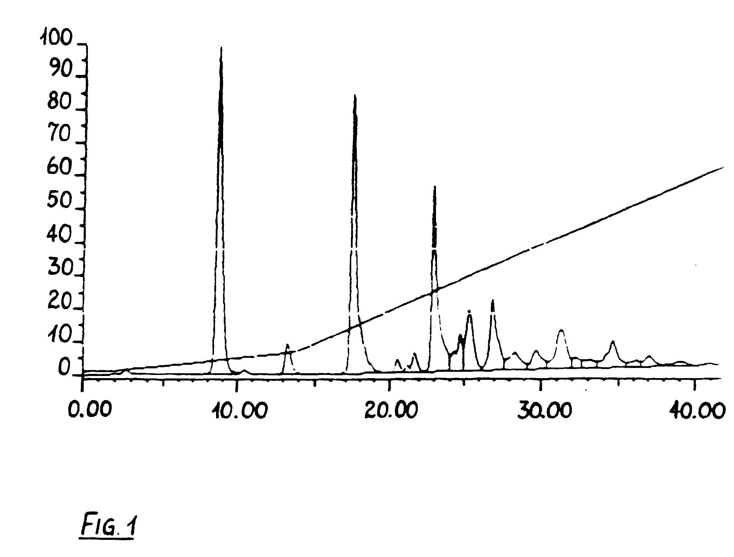

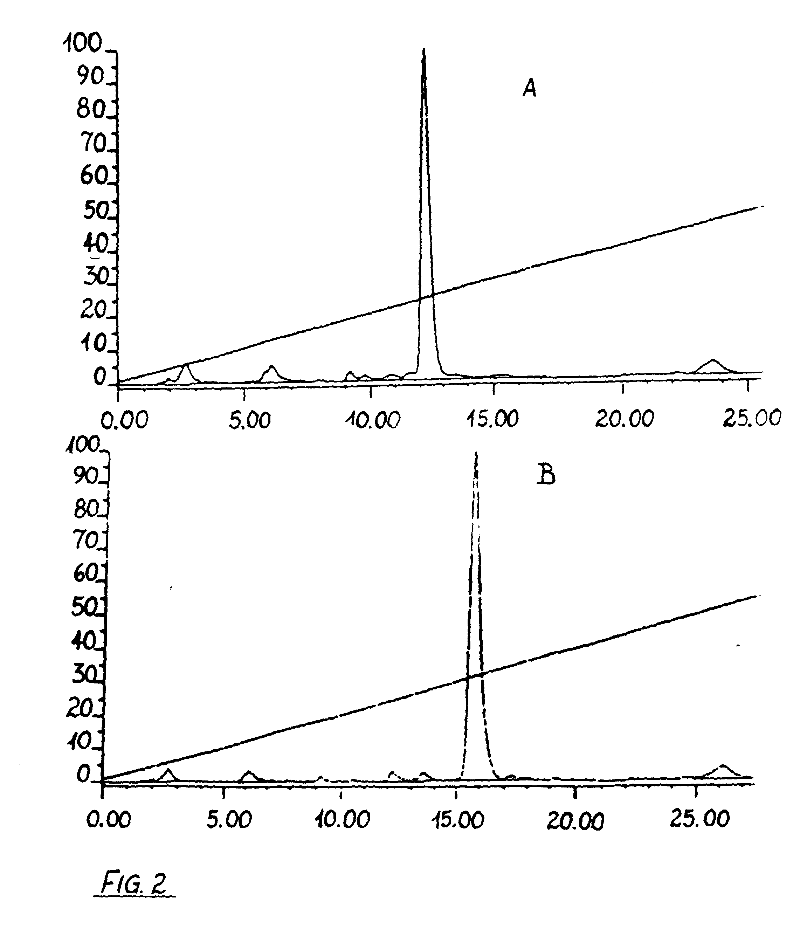

Sämtliche Reaktionen werden bei Raumtemperatur durchgeführt.
a) Synthese von Poly(A):
   0,9 g (2,5 mMol) Adenosin-2'(3')-monophosphat in H⁺-Form und 1,2 ml (2,6 mMol) tri-n-Octylamin werden in 30 ml Methanol/Ethanol (1:1) gelöst, wobei die Lösung mehrere Stunden gerührt wird. Danach werden die unlöslichen Anteile mit Hilfe eines Glasfilters abfiltriert, wonach das Filtrat mit Hilfe eines Rotationsverdampfers bis zur Trockene eingedampft wird. Der erhaltene Feststoff wird dann in 10 ml abs. Dioxan gelöst und mit Hilfe eines Rotationsverdeampfers bis zur Trockene eingedampft. Beide Vorgänge werden zweimal wiederholt. Dann wird der erhaltene Feststoff in 5 ml abs. Dioxan gelöst, wonach 0,77 ml (3,75 mMol) Diphenylphosphorylchlorid unter Rühren mit Hilfe eines Magnetrührers zugetropft werden. Danach werden 3,3 ml (7,5 mMol) tri-n-Octylamin zugesetzt. Dann wird die erhaltene Lösung 1 Stunde lang gerührt, wonach 0,77 ml Diphenylphosphorylchlorid und 3,2 ml tri-n-Octylamin zugesetzt werden und weitere 4 Stunden gerührt wird. Dann wird das Reaktionsgemisch in 5 Vol. Hexan/Ether (4:6) unter Rühren gegossen. Der Niederschlag wird dann mit Hilfe eines Glasfilters abfiltriert und mit dem erwähnten Gemisch und danach mit Ether gewaschen und im Vakuum getrocknet. Man erhält auf diese Weise ein weißes Pulver von Polyadenylat mit unregelmäßigen 2',5'- und 3',5'-Internucleotidbindungen. Die allgemeine Formel des Gemisches ist (A2'(3')p-)ₙ A>p, worin n = 10.
   Das trockene Polymergemisch wird in 12 ml Wasser gelöst, indem man den pH durch Zugabe von konz. Ammoniaklösung auf 9,3 einstellt. Danach werden 3 Vol. Ethanol zugegeben, worauf der pH mit 5 M HCl auf 3,0 eingestellt wird. Der nach Halten des Gemisches bei -18°C gebildete Niederschlag wird abzentrifugiert, mit 75 % Ethanol gewaschen und im Luftstrom getrocknet. Ein erheblicher Anteil des Monomers bleibt im Überstand zurück.
b) Selektive Spaltung der 3',5'-Internucleotidbindungen durch Spaltung mit Binase
   Der in a) erhaltene Niederschlag wird in 40 ml H₂O gelöst, wobei der pH mit einer 3 M-Lösung von tris-Base auf 7,0 eingestellt wird, wonach 2 ml einer Lösung von RNAse aus Bacillus intermedius (Binase, E.C.3.1.4.23) (150 E/mg) zugesetzt werden und dann während 10 bis 12 Stunden gerührt wird. Danach wird das Enzym durch Extraktion mit demselben Volumen an Chloroform/Isoamylalkohol (24:1) extrahiert. Die erhaltene wässerige Phase enthält ein Gemisch aus 2',3'-Cyclophosphaten von Adenosin und 2',5'-Oligoadenylaten.
c) Isolierung der Einzelverbindungen
   Die vorgängige Abtrennung der einzelnen Komponenten erfolgt durch Ionenaustauschchromatographie mit Hilfe von DEAE Spheron 1000 in HCO₃-Form (16 x 600 mm, 120 ml). Das enzymatische Spaltprodukt von Poly(A) wird nach Deproteinierung durch ca. 8- bis 10malige Rotationsverdampfung eingeengt und nach Zugabe von zehn Vol. Ethanol bei -18°C während 12 Stunden gehalten. Nach Zentrifugieren (10 bis 20 min, 3000 U/min) wird der Niederschlag in Wasser gelöst und auf die Säule (optische Dichte 25.000 bis 50.000 E bei 260 nm) aufgegeben. Nach Waschen der Säule mit Wasser und mit 0,05 M Triethylammoniumbicarbonat (pH 8,0) werden die Produkte bei linearem Gradienten dieses Salzes (0,1 bis 0,8 M, Gesamtvolumen 1 L) eluiert. Die die angereicherten Einzelverbindungen enthaltenden Fraktionen werden dann am Rotationsverdampfer bis zur Trockene eingedampft und zur vollständigen Entfernung der Pufferkomponenten lyophilisiert.
   Die abschließende Reinigung der individuellen Verbindungen erfolgt durch HPLC, wie unten angegeben (Beschickung der halbpräparativen Säule mit ca. 2.000 E optische Dichte). Die Entsalzung der Lösung nach HPLC erfolgt durch Sorption einer 10- bis 20fach verdünnten Lösung an kleinen DEAE-Säulen nach Waschen mit Wasser, Eluierung mit einem geringen Volumen von 1 M Triethylammoniumbicarbonat und Lyophilisierung der Lösung.
d) Reinigung und Analyse der 2',5'-Oligoadenylate durch HPLC-Chromatographie
   Die Ionenaustauschchromatographie erfolgt an einer NH₂-Diasorb-Säule (8 µm, 4 x 150 mm für die Analyse und 10 µm, 9,2 x 250 mm für die abschließende präparative Reinigung); linearer Gradient: Eluent A - 20 % MeOH; B - 2M AmAc, 20 % MeOH, 2 % B pro Gemisch; Fließgeschwindigkeit - 0,7 ml/min für die analytische Säule und 4 ml/min für die präparative (Fig. 1 bis 3). Der Nachweis erfolgt mit Hilfe eine UV-Überwachungsgeräts bei 260 nm.
   Die Gemische enthalten aufgrund der optischen Dichte bei 260 nm 40 bis 50 % Monomer, ca. 20 bis 30 % Diadenylat, ca. 5 bis 15 % Triadenylat, 2 bis 8 % Tetraadenylat und 2 bis 7 % höhere Oligoadenylate. Der durchschnittliche molare Extinktionskoeffizient betrug 15.10³ pro Adenosinrest, 36,8.10³ für das Trimer und 45,8.10³ für das Tetramer.
e) Bestimmung der Zusammensetzung der Einzelverbindungen.
   Jede Verbindung wurde zur Öffnung der Cyclophosphatgruppe bei pH 1,0 (1 Std. bei 37°C) gehalten, dann durch bakterielle alkalische Phosphatase dephosphoryliert und der Alkalihydrolyse der Internucleotidbindungen (0,3 M NaOH, 48 Stunden bei 20°C) unterworfen. Das Verhältnis der Endprodukte (Adenosin zu Adenosin-2'(3')-phosphat) wurde durch HPLC ermittelt. Die dabei erzielten Ergebnisse stimmen mit den vorhergesagten gut überein: Ado:AMP = 1:1 für das Dimer, 1:2 für das Trimer und 1:3 für das Tetramer. Der Standardfehler lag unter 5 %. Die durch Säureöffnung der 2',3'-Cyclophosphatgruppe erhaltenen Verbindungen waren aufgrund ihrer HPLC-Beweglichkeit und NMR-Spektra (³¹P und ¹H) identisch mit den bekannten 2'(3')-Phosphaten der entsprechenden Oligoadenylate. Andererseits werden die erhaltenen 2'(3')-Phosphate quantitativ in die Ausgangs-2',3'-Cyclophosphate der entsprechenden Oligoadenylate durch Einwirkung von BrCN (100facher molarer Überschuß) in wässeriger Lösung bei Raumtemperatur quantitativ umgesetzt.
f) Ermittlung des Internucleotid-Bindungstyps
   Die Behandlung der 2',3'-Cyclophosphate der einzelnen 2',5'-Oligoadenylate mit frischer Binase bewirkt unter Bedingungen, die zu einer vollständigen Aufspaltung zu Polyadenylsäure - Adenosin - 2',3'-Cyclophosphat führen, keinerlei Veränderung im HPLC-Abtrennungsprofil und in den ³¹P NMR-Spektra.
   In den ³¹P NMR-Spektra wurden lediglich die Peaks festgestellt, die der endständigen Cyclophosphatgruppe (20 bis 21 ppm) und dem 2',5'-Internucleotidphosphat (0,02 bis 0,4 ppm) entsprachen, und zwar bei einem Verhältnis von 1:1 fur das Dimer, 1:2 für das Trimer und 1:3 für das Tetramer. Der Standardfehler lag unter 2 %. Die 3',5'-Bindung (unter 1 % Internucleotidphosphat) wurde nur für das Tetramer nachgewiesen.
   Die Oligoadenylate sind farblose Verbindungen und in Wasser (bis zu 10⁻² M), Dimethylsulfoxid, wässerigem Ethanol oder Glycerol leicht löslich. Sie sind in neutraler wässeriger Lösung bei 4°C mehrere Monate lang und in eingefrorener Lösung (-20°C) oder in lyophilisiertem Zustand unbegrenzt löslich.

### Pharmakologische Beispiele

Die klinische Untersuchung des pharmazeutischen Präparats erfolgte an Papillomatose-Patienten. Die Behandlung erfolgte mit dem Na- oder Triethylammoniumsalz der entsprechenden Verbindung, die auf die erforderliche Konzentration mit Wasser oder 0,1 M NaCl verdünnt worden war. Die Konzentration der wirksamen Verbindung in der Endlösung wurde anhand ihrer optischen Dichte bei 260 nm unter Heranziehung der oben angeführten molaren Extinktionskoeffizienten ermittelt. Die dabei erzielten Ergebnisse sind nachfolgend zusammengefaßt. Die Beispiele illustrieren lediglich die Erfindung, schränken sie jedoch nicht ein.

### Beispiel 1

### Klinische Prüfung von 2',5'-Triadenylat-2',3'-cyclophosphat (n = 1, AIII)

Diagnose bei Patientin M. (1958): scharfkantige Kondylome. Befund: im Perineum-Bereich vier Kondylome (d = 1 bis 2 mm, h = 2 bis 8 mm), die hautfarben, rauh, elastisch und manchmal schmerzhaft waren. Begleiterkrankung: Kolpitis.

Applikation von AIII (10⁻⁴ M) bei täglich ca. 50 µl pro Kondylom. Am 4. Tag zeigten sämtliche Kondylome eine runzelige Oberfläche und am 8. Tag waren sie vollständig verschwunden. Nach 8 Wochen waren keine Rückfälle zu beobachten.

Diagnose bei Patientin P. (1950): mehrfache Kondylome. Befund: im Perineum-Bereich 14 Kondylome (d = 2 bis 8 mm, h = 2 bis 5 mm), die elastisch, hautfarben und manchmal schmerzhaft waren.

Applikation von AIII (10⁻⁴ M) während 2 Wochen täglich 50 µl pro Kondylom. Am 6. Tag waren sämtliche Kondylome auf die Hälfte bis ein Drittel geschrumpft und am 10. Tag waren 8 Kondylome vollständig verschwunden, ohne auf der Haut Spuren zu hinterlassen. Die übrigen 6 Kondylome waren weiterhin vorhanden und nahmen 3 bis 4 Wochen nach Beendigung der Behandlung wieder ihre ursprüngliche Größe an. Die Behandlung wurde nicht wiederholt. 10 Wochen nach Ende der Behandlung waren keine Veränderungen festzustellen (keine Rückfälle bzw. keine Bildung neuer Kondylome).

Diagnose bei Patientin C (1958): Sohlenwarze. Befund: Eine Warze auf der Unterseite der großen Zehe (d = 5 mm, h = 3 mm), die kompakt, von der Farbe der umgebenden Gewebe, mit rauher Oberfläche, beim Gehen stark schmerzend war.

Applikation von AIII (10⁻⁴ M) täglich 50 µl pro Warze. Am 6. Tag war die Warze weicher geworden und bei mechanischer Einwirkung ließ sich die Warzenoberfläche wegreiben. Am 10. Tag war die Warze vollständig verschwunden und beim Gehen waren keine Schmerzen mehr zu verspüren. Bei der Kontrolle nach 12 Wochen zeigten sich keine Rückfälle. Die betreffende Stelle der Hautoberfläche unterschied sich in nichts von dem umgebenden Gewebe.

### Diagnose bei Patientin C. (1926): Papillome.

Befund: Auf der Vorderseite und auf der rechten Seite des Halses wurden 7 Papillome festgestellt, und zwar zwei große (d = 3 und 2 mm, h = 2,5 mm, dunkelbraun) und 5 kleinere (d = 0,8 bis 1,5 mm, h = 0,8 bis 1,5 mm, hautfarben). Aufgetreten sind die Papillome 1982 während der Menopause.

Applikation von AIII (10⁻⁵ M), während 2 Wochen täglich ca. 50 µl pro Papillom. Am 5. Tag waren die kleineren Papillome verblaßt, geschrumpft und abgeflacht. Weitere Veränderungen waren nicht festzustellen. 2 bis 3 Wochen nach Beendigung der Behandlung wiesen die Papillome wieder die ursprüngliche Form und Größe auf.

### Beispiel 2

### Klinische Prüfung von 2',5'-Tetraadenylat-2',3'-cyclophosphat (n = 2, AIV)

Diagnose bei Patientin S, 27 Jahre alt: gewöhnliche Warzen. Befund: Drei Warzen auf dem Bauch, 3 cm unterhalb des Nabels von gräulicher Farbe, vorstehend, Durchmesser 2 bis 3 mm. Die Warzen wurden zwei Wochen lang mit 1 bis 3 Tropfen AIV (10⁻⁵ M) täglich behandelt. Am 7. bis 10. Tag nach Beginn der Behandlung waren alle Warzen geschrumpft und abgeflacht. Drei Wochen nach Ende der Behandlung war eine der Warzen völlig verschwunden und vier Wochen nach Ende der Behandlung alle übrigen. Keine narbenähnlichen Veränderungen in der Haut.

Sieben weitere Patienten mit mehrfachen Warzen, und zwar mit Kondylomen (sechs Patienten), gewöhnlichen Warzen (drei Patienten) und nicht identifizierten Warzen (vier Patienten) wurden mit einer wässerigen Lösung (10⁻⁵ M) von AIV täglich zwei Wochen lang behandelt. Die Kondylome befanden sich an den äußeren Genitalien und auf der Brust, die Warzen an den Handgelenken, am Körper und an den Beinen.

In den meisten Fällen schrumpften und verkleinerten sich die Kondylome und Warzen (insbesondere erstere) nach sechs- bis neuntägiger Behandlung. 1 bis 2 Wochen nach Ende der Behandlung oder während dieser Zeit waren drei Patienten bereits völlig kondylomenfrei und zwei zeigten keine gewöhnlichen Warzen mehr. Ein Patient war ca. 6 Wochen nach Beendigung der Behandlung völlig frei von sämtlichen sechs Kondylomen unter der Achselhöhle. In zwei Fällen verschwanden während oder nach der Behandlung die kleinen Kondylome (d < 3 mm, insgesamt 11) an den äußeren Genitalien, während die größeren im selben Bereich (d > 5 mm, zwei im ersten Fall und drei im zweiten) 2 bis 6 Wochen nach der Behandlung ihre ursprüngliche Größe und Form wiedererlangten. Zwei der sechs nicht identifizierten Warzen waren 2 bis 3 Wochen nach der Behandlung verschwunden, die übrigen zeigten jedoch keine Reaktion.

In keinem einzigen Fall wurden während der Behandlung oder 8 bis 12 Wochen danach negative Reaktionen, Spuren auf der Haut oder Rückfälle beobachtet.

### Beispiel 3

### Klinische Prüfung des Gemisches A (Gemisch von 2',5'-Oligoadenylat-2',3'-cyclophosphaten, n ≥ 0)

Getestet wurden zwei Patienten, und zwar einer mit drei gewöhnlichen Warzen (3 bis 4 mm) am Hals, und ein zweiter mit zwei Kondylomata acuminata auf der Innenseite des Beins. Jede Warze wurde täglich mit 1 bis 2 Tropfen 10⁻⁴ M wässeriger Lösung des Gemisches A befeuchtet. Am 9. Behandlungstag waren sämtliche gewöhnlichen Warzen verschwunden. Ein kleines Kondylom verschwand nach 7 Tagen Behandlung, ein anderes drei Wochen nach Beendigung der Behandlung, d.h. fünf Wochen nach Beginn der Behandlung.

Die erzielten Ergebnisse zeigen deutlich die hohe Wirksamkeit der untersuchten Verbindungen und des Verfahrens zur Behandlung von durch menschliche Papillomaviren verursachten Haut- und Epithelläsionen. In keinem einzigen Fall war die Behandlung der Papillomatosen mit 2',5'-Oligoadenylaten von einer schmerzhaften oder entzündlichen Reaktion sowie einer subjektiven oder objektiven Verschlechterung des Zustandes des Patienten begleitet.

Die hohe klinische Wirksamkeit der 2',5'-Oligoadenylate bei sehr geringer Dosierung und das Fehlen lokaler Reaktionen und systemischer Nebenwirkungen sowie die Rezidivfreiheit beweisen die Vorteile der genannten Verbindungen für die Behandlung von Papillomatosen.

Ähnliche Ergebnisse wie bei der Behandlung von Papillomatosen wurden auch bei Verwendung bereits bekannter Verbindungen, d.h. von 2',5'-Oligoadenylaten mit natürlichen Adenosinresten und 2'(3')-Phosphatgruppen (Serie B) oder freien 2'- und 3'-Hydroxylgruppen (Serie C) am 3-endständigen Adenosinrest erzielt. In diesen Fällen erweisen sich bei der Behandlung äußerer Papillomatosen sowohl die Einzelverbindungen, wie z.B. Trimere und Tetramere oder ihre Gemische mit anderen Oligomeren Trimere B und C, Tetramere B und C, Gemische B und C) als überaus wirksam.

### Erläuterung der Zeichnungen:

Fig 1:
   HPLC-Analyse des Gemisches A (2',5'-Oligoadenylat-2',3'-cyclophosphate). Säule: Diasorb NH₂, 4x150 mm.
   Mobile Phase: A - 20 % CH₃OH; B - 2M AcONH₄ in 20 % CH₃OH. Fließgeschwindigkeit: 0,7 ml/min; 0'-2' - 1 % B, 2'-14'-0,5 % B/min, 14'-50' - 2 % B/min.
   Nachweis bei 260 nm. Zusammensetzung des Gemisches entsprechend der Absorption bei 260 nm: 23 % Monomer (8,7 min), 22,5 % Dimer (17,5 min), 14 % Trimer (23 min), 6,8 % Tetramer (26,8 min).
Fig. 2:
   HPLC-Analyse der gereinigten 2',5'-Trimer-(A)- und Tetramer-(B)-2',3'-cyclophosphate.
   Säule, Zusammensetzung der mobilen Phase und Fließgeschwindigkeit wie in Fig. 1, Gradient - 2 % B/min.

## Claims

1. A pharmaceutical composition comprising one or more 2',5'- oligoadenylate-2',3'-cyclophosphates having the formula: 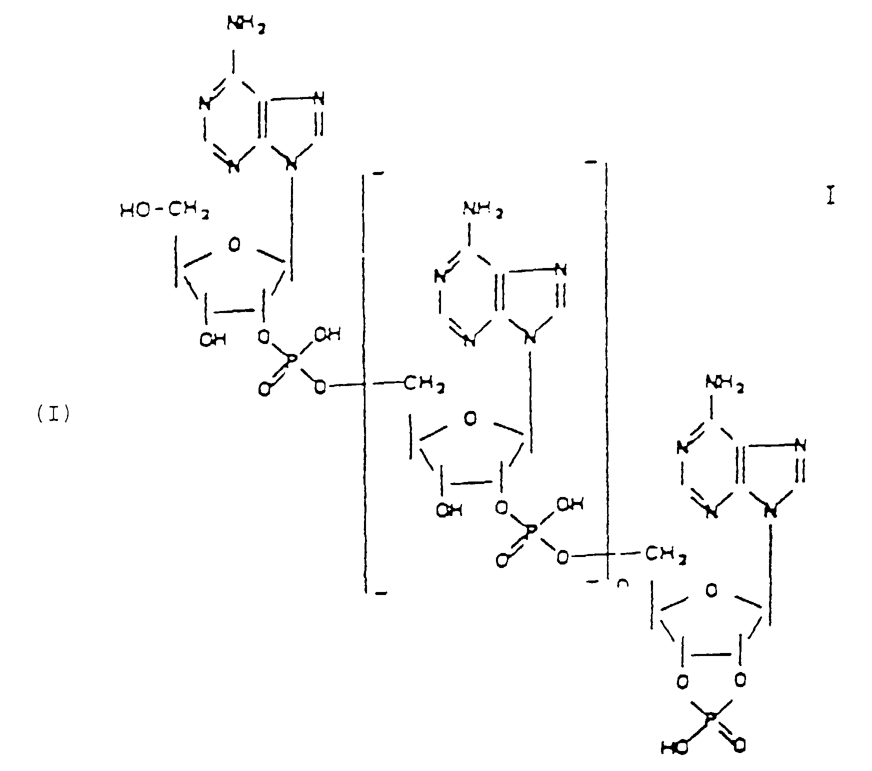 wherein 0≤ n ≤ 10 is a mixture with a pharmaceutically acceptable carrier or solvent.

2. The pharmaceutical composition of claim 1, wherein composition contains between 10⁻⁴ to 10⁻⁷M of the compound as active ingredient.

3. A 2',5'-oligoadenylate-2',3'-cyclophosphate having the formula: 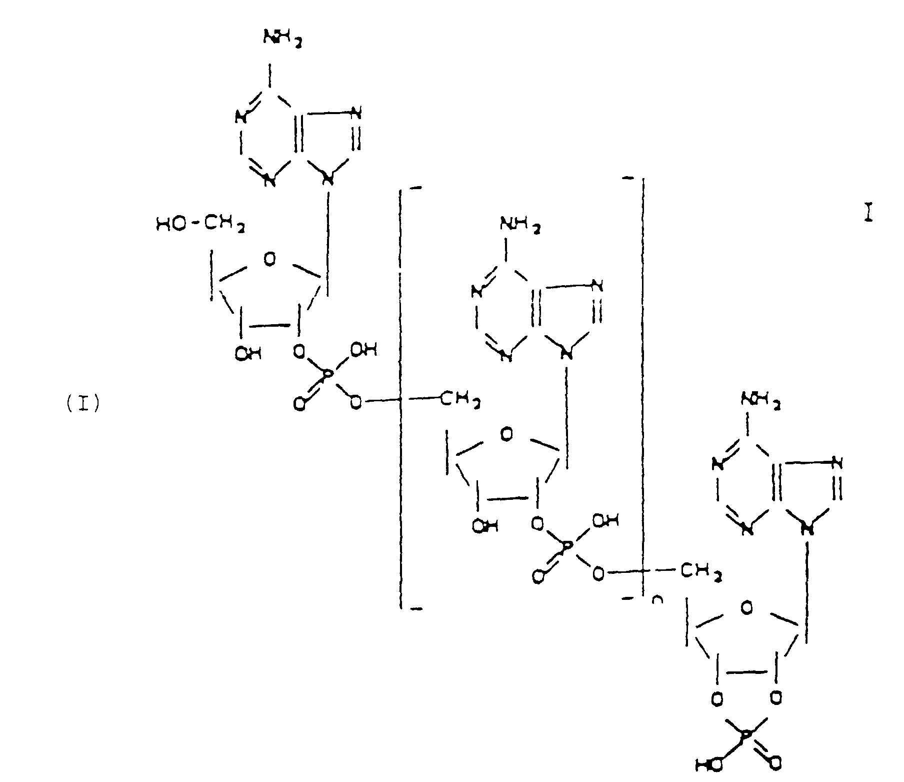 wherein 0 ≤ n ≤ 0 for pharmaceutical use.

4. Use of a 2',5'-oligoadenylate-2',3'-cyclophosphate having the formula: 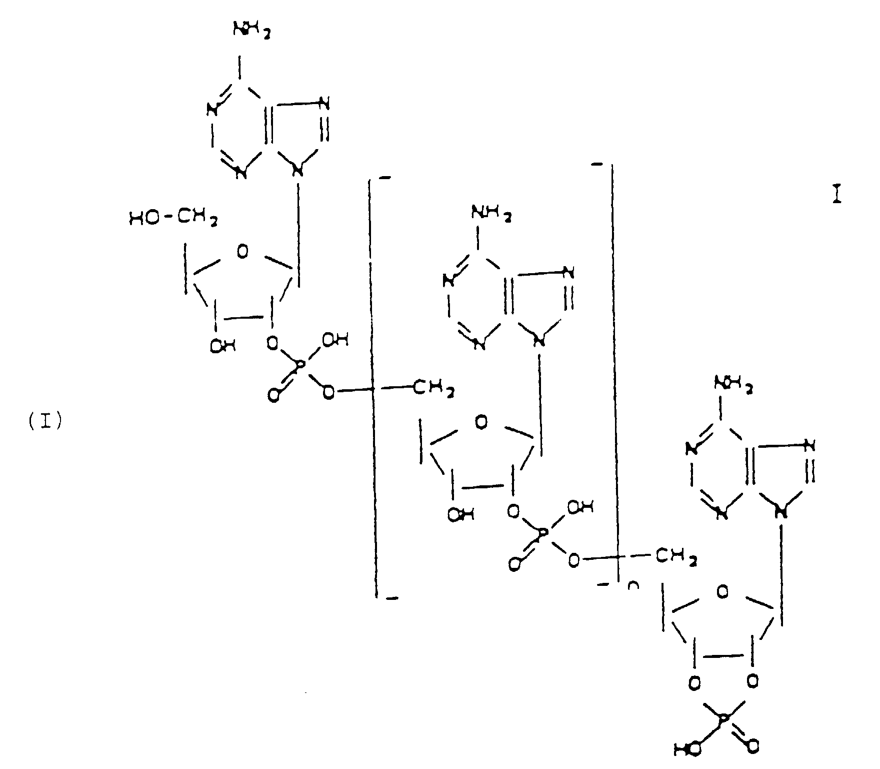 wherein 0≤ n ≤ 10, for the preparation of a medicament for the treatment of external papillomatoses.

5. Use of a 2',5' oligoadenylate for the preparation of a medicament for the treatment of external papillomatoses.

6. The use of claim 4 or claim 5, wherein the condition is benign or precancerous, skin or mucosal lesions.

7. The use of claim 4 or claim 5, wherein the condition is papillomatoses.

## Patentansprüche

1. Pharmazeutisches Präparat, das eine der 2',5'-Oligoadenylat-2',3'-cyclophosphate der allgemeinen Formel: 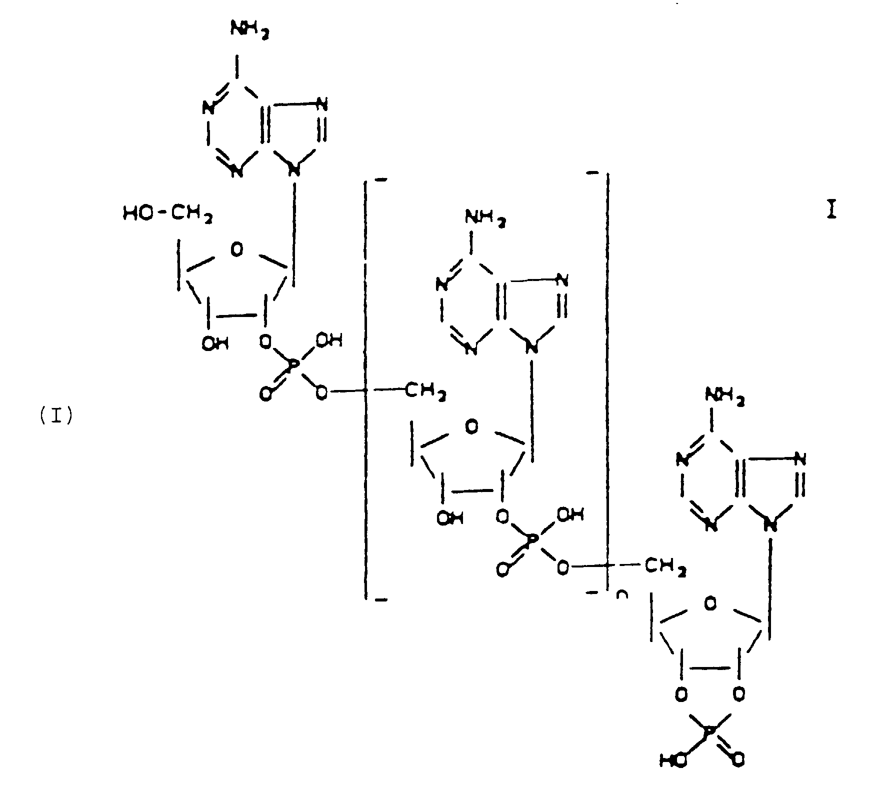 worin 0 ≤ n ≤ 10, oder Gemische davon als Wirkstoff im Gemisch mit einem pharmazeutisch verträglichen Träger oder Lösungsmittel enthält.

2. Pharmazeutisches Präparat nach Anspruch 1, worin die Wirkstoffkonzentration beträgt 10⁻⁴ bis 10⁻⁷ M.

3. 2',5'-Oligoadenylat-2',3'-cyclophosphate der allgemeinen Formel: 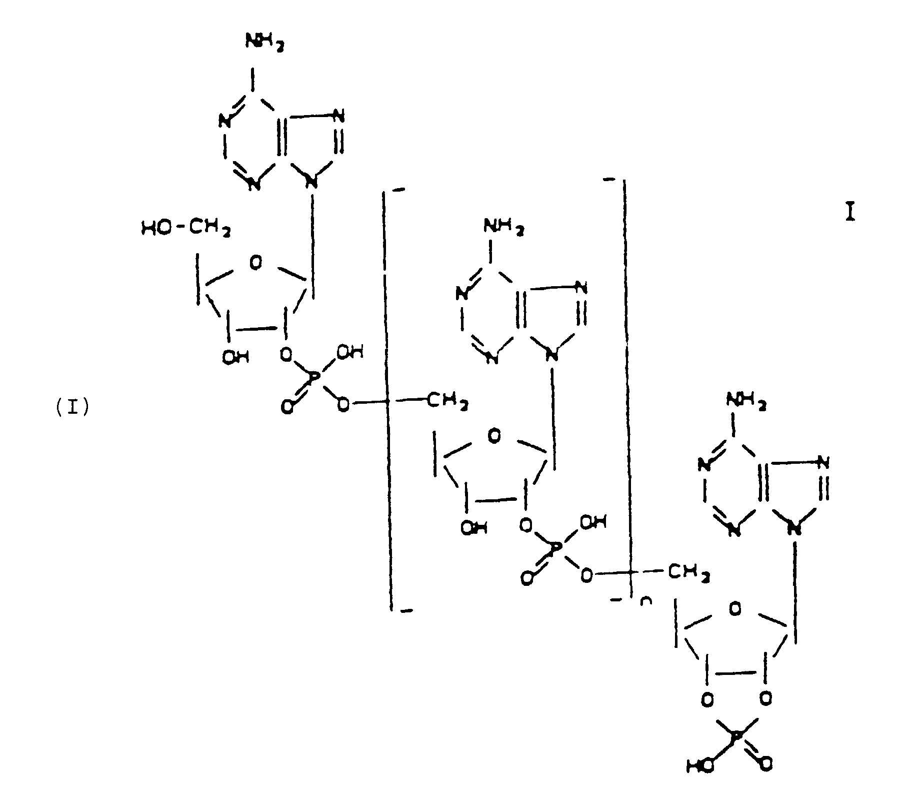 worin 0 ≤ n ≤ 10, zur pharmazeutischen Verwendung.

4. Verwendung von eine 2',5'-Oligoadenylat-2',3'-cyclophosphate der allgemeinen Formel: 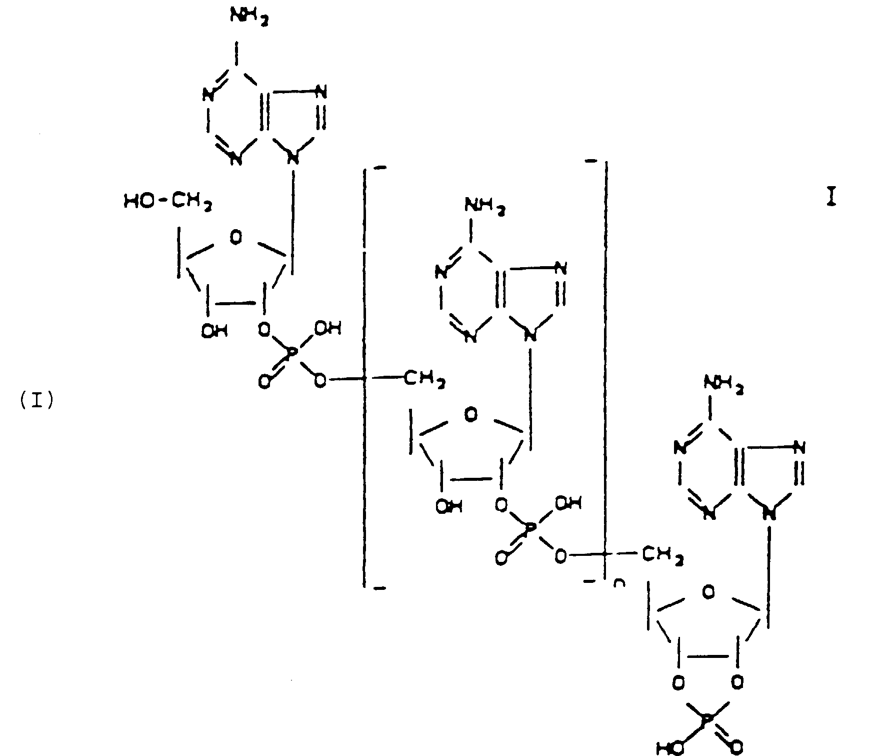 worin 0 ≤ n ≤ 10, zur Herstellung eines Medikaments zur Behandlung äußerer Papillomatosen.

5. Verwendung von eine 2',5' Oligoadenylat, zur Herstellung eines Medikaments zur Behandlung äußerer Papillomatosen.

6. Verwendung nach Anspruch 4 oder 5, worin die Beschwerde gutartiger oder präkanzeröser Haut oder Epithelläsionen ist.

7. Verwendung nach Anspruch 4 oder 5 worin die Beschwerde Papillomatosen ist.

## Revendications

1. Préparation pharmaceutique qui contient un des 2',5'-oligoadénylate-2',3'-cyclophosphates répondant à la formule générale: 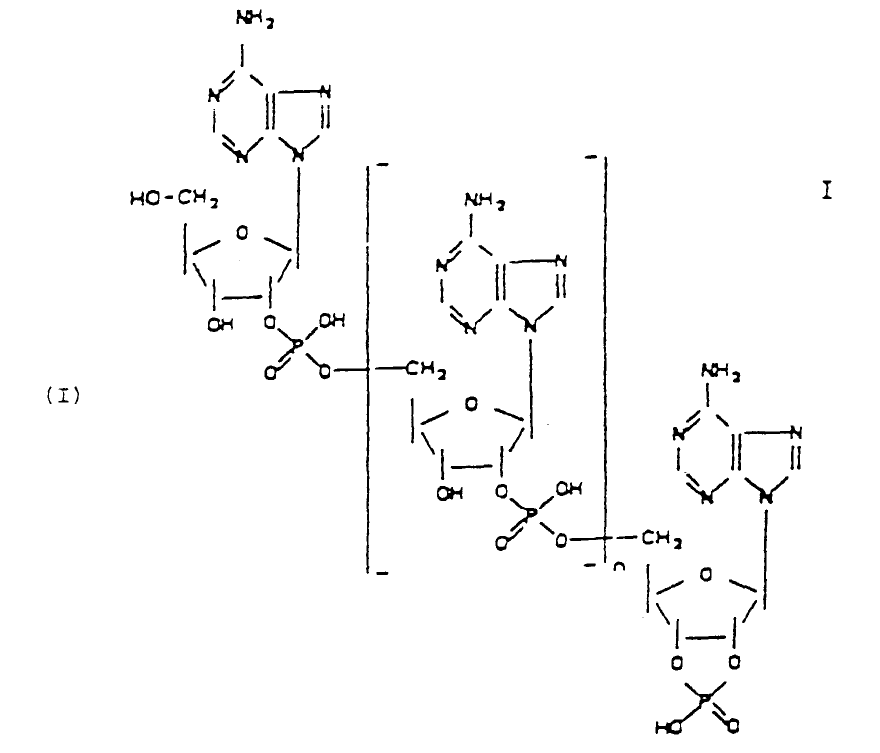 dans laquelle 0 ≤ n ≤ 10, ou leurs mélanges à titre de principe actif en mélange avec un support ou un solvant pharmaceutiquement acceptable.

2. Préparation pharmaceutique selon la revendication 1, dans laquelle la concentration du principe actif s'élève de 10⁻⁴ à 10⁻⁷ M.

3. 2',5'-oligoadénylate-2',3'-cyclophosphate répondant à la formule générale: 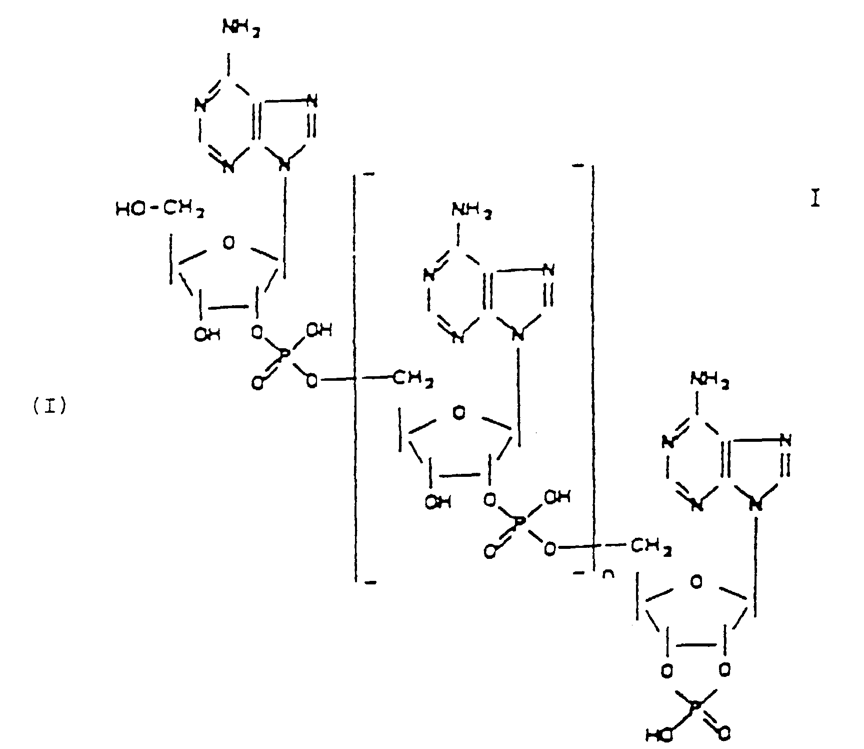 dans laquelle 0 ≤ n ≤ 10, à des fins d'utilisation pharmaceutique.

4. Utilisation d'un 2',5'-oligoadénylate-2',3'-cyclophosphate répondant à la formule générale: 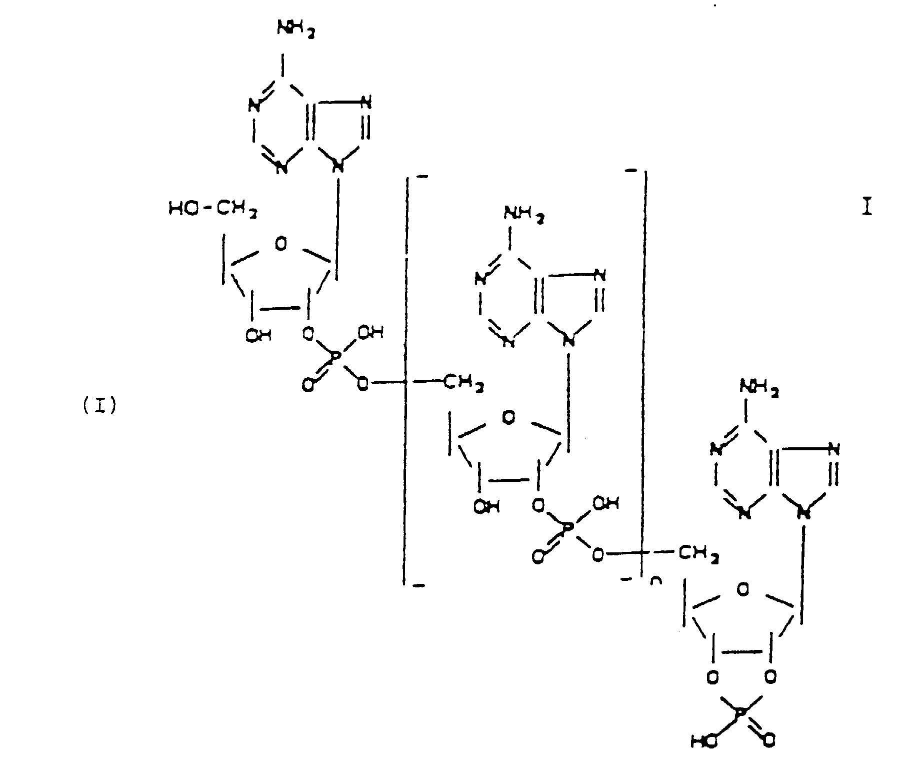 dans laquelle 0 ≤ n ≤ 10, pour la préparation d'un médicament destiné au traitement des papillomatoses externes.

5. Utilisation d'un 2',5'-oligoadénylate pour la préparation d'un médicament destiné au traitement de papillomatoses externes.

6. Utilisation selon la revendication 4 ou 5, dans laquelle le problème de santé concerne des lésions cutanées ou épithéliales bénignes ou précancéreuses.

7. Utilisation selon la revendication 4 ou 5, dans laquelle le problème de santé concerne des papillomatoses.
